# EUROPEAN PATENT APPLICATION

(11) **EP 4 700 121 A1**
(43) Date of publication of application: **25.02.2026**
(21) Application number: 24792955.7
(22) Date of filing: 15.04.2024
(51) Int. Cl.: C12N 9/88, C12N 15/70, A23K 10/16

(54) **NOVEL POLYPEPTIDE EXHIBITING DON-DEGRADING ACTIVITY**

(30) Priority: 17.04.2023 KR 20230049991
(71) Applicant: CJ Cheiljedang Corporation, Seoul 04560 (KR)
(72) Inventor: LEE, Seung-hee, Seoul 04560 (KR); KIM, Nam Yoon, Seoul 04560 (KR); YANG, Tae Joo, Seoul 04560 (KR); HONG, So Yeon, Seoul 04560 (KR); KANG, Young Seo, Seoul 04560 (KR)
(74) Representative: Meissner Bolte Partnerschaft mbB
(86) International application number: PCT/KR2024/004996
(87) International publication number: WO 2024/219769

(57) **Abstract**

The present disclosure relates to a modified polypeptide having DON degradation activity.

## Description

### [Technical Field]

The present disclosure relates to a modified polypeptide having deoxynivalenol (DON) degradation activity.

### [Background Art]

Mycotoxins are a collective term for fungal-derived metabolites that are toxic to animals. When crops or stored agricultural products are infected or contaminated with fungi that produce mycotoxins, the generated mycotoxins can pose health risks to animals or humans. Trichothecene, which is often found in grains, is a sesquiterpenoid mycotoxin having a 12,13-epoxy-trichothec-9-ene structure and is mainly produced by *Fusarium, Myrothecium, Stachybotrys,* and *Trichothecium.* From an economic standpoint, the most important trichothecene-producing genus is *Fusarium,* and among these toxins, the ones most frequently detected in grains are deoxynivalenol (DON), nivalenol (NIV), and their acetylated derivatives.

Trichothecenes act on eukaryotic cells to exhibit various toxic effects such as inhibition of protein synthesis, cytotoxicity, and cell death. Ingestion of feed or food contaminated with DON can cause symptoms such as immunosuppression, anemia, headache, nausea, and abdominal pain in humans, and symptoms such as feed refusal, vomiting, growth inhibition, and reproductive disorders in animals. Due to the impact of global warming, the annual level of exposure of humans or animals to trichothecenes is expected to exceed tolerable levels.

Most countries have legally established allowable limits for DON detection in feed, food, and harvested grains. Attempts have been made to reduce DON contamination by using pesticides targeting *Fusarium* spp. or through breeding; however, DON still contaminates grains. To detoxify DON, various chemicals such as ozone, ammonia, chlorine, hydrogen peroxide, and sodium bisulfite have been used; however, these have not been scaled up due to cost, safety concerns, and adverse effects on grain quality. The most effective method is the treatment with sodium metabisulfite; however, in Europe, its application to food-use grains is prohibited, creating a need for a safe method of detoxifying DON.

### [Prior Art Documents]

### [Non-patent Documents]

(Non-patent document 1) S Chakraborty et al., Virulence. Jan 2015; 6(1): 50-65 (2014.12.17), Lactoylglutathione lyase, a critical enzyme in methylglyoxal detoxification, contributes to survival of Salmonella in the nutrient rich environment.

### [Disclosure]

### [Technical Problem]

The problem to be solved by the present disclosure is to provide a modified polypeptide having deoxynivalenol (DON) degradation activity.

### [Technical Solution]

An object of the present disclosure is to provide a modified polypeptide having deoxynivalenol (DON) degradation activity.

Another object of the present disclosure is to provide a polynucleotide encoding the modified polypeptide of the present diclosure.

Still another object of the present disclosure is to provide a microorganism comprising one or more of: the modified polypeptide; a polynucleotide encoding the modified polypeptide; and a vector comprising the polynucleotide.

Still another object of the present disclosure is to provide a composition for degrading DON, comprising one or more of: the modified polypeptide; and a microorganism comprising one or more of the modified polypeptide, a polynucleotide encoding the modified polypeptide, and a vector comprising the polynucleotide.

Still another object of the present disclosure is to provide a feed additive composition, comprising one or more of: the modified polypeptide; and a microorganism comprising one or more of the modified polypeptide, a polynucleotide encoding the modified polypeptide, and a vector comprising the polynucleotide.

Still another object of the present disclosure is to provide a method for degrading DON, comprising reacting DON with one or more of: the modified polypeptide; and a microorganism comprising one or more of the modified polypeptide, a polynucleotide encoding the modified polypeptide, and a vector comprising the polynucleotide.

Still another object of the present disclosure is to provide a method for preparing a feed product, comprising mixing a feed ingredient with a feed additive composition comprising one or more of: the modified polypeptide; and a microorganism comprising one or more of the modified polypeptide, a polynucleotide encoding the modified polypeptide, and a vector comprising the polynucleotide.

Still another object of the present disclosure is to provide a method for preparing a modified polypeptide having DON degradation activity, comprising culturing a microorganism comprising one or more of: the modified polypeptide, a polynucleotide encoding the modified polypeptide, and a vector comprising the polynucleotide.

### [Advantageous Effects]

The modified polypeptide having DON degradation activity of the present disclosure has improved thermal tolerance and thermal stability, and can be usefully applied in various industrial fields.

### [Brief Description of the Drawings]

FIG. 1 is a diagram confirming the sequence homology of a polypeptide (GbSPG-7P; SEQ ID NO: 1) having DON degradation activity with a modified polypeptide of the present disclosure.
FIG. 2 is a diagram confirming the effect of improved DON degradation activity of modified polypeptides of the present disclosure compared to the polypeptide (GbSPG-7P; SEQ ID NO: 1) having DON degradation activity.
FIG. 3 is a diagram confirming the effect of improved thermal stability or thermal tolerance of modified polypeptides of the present disclosure compared to the polypeptide (GbSPG-7P; SEQ ID NO: 1) having DON degradation activity.
FIG. 4 is a diagram confirming the effect of improved DON degradation activity of modified polypeptides of the present disclosure.
FIG. 5 is a diagram confirming the effect of improved thermal stability or thermal tolerance of modified polypeptides of the present disclosure.
FIG. 6 is a diagram confirming the effect of improved DON degradation activity of a modified polypeptide of the present disclosure.
FIG. 7 is a diagram confirming the effect of improved thermal stability or thermal tolerance of a modified polypeptide of the present disclosure.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below.

Furthermore, those skilled in the art may be able to recognize or identify numerous equivalents to the particular aspects of the present disclosure described herein by using routine experimentation. Moreover, these equivalents are intended to be included in the present disclosure.

Additionally, a number of papers and patent documents have been cited throughout the present specification. The content of the cited papers and patent documents is incorporated herein by reference in their entirety to describe the level of the technical field to which the present disclosure belongs and the contents of the present disclosure more clearly.

As used in the specification and appended claims of the present disclosure, the singular articles ("a", "an", and "the") include plural referents unless the context clearly indicates otherwise. Further, unless the context indicates otherwise, singular terms include their plural forms, and plural terms include their singular forms. As used in the specification and appended claims of the present disclosure, the use of "or" may include the meaning of "and/or", unless indicated otherwise.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. For example, the term "about" can refer to a range of -10% to +10% of the numerical value. As another example, the term "about" may refer to a range of -5% to +5% of the given numerical value. But it is not limited thereto.

As used herein, terms such as "first, second, third, ...", "i), ii), iii), ...", or "(a), (b), (c), (d), ..." are used to distinguish similar elements and do not imply that the elements are executed continuously or in the listed order. For example, when these terms are used in relation to a method, use, or analytical step, the steps may be performed without any time intervals, simultaneously, or with intervals of several seconds, minutes, hours, days, or months.

As used herein, the term "consisting essentially of" may mean that, in the case where the features of the matter claimed herein are not substantially affected by the presence of an unspecified component, the unspecified component may be present.

As used herein, the term "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising" refers to the presence of features, steps, or components following the term and does not exclude the presence or addition of one or more other features, steps, or components. As used herein, the components or features following the term "comprising" may be essential or mandatory; however, in certain embodiments, other optional or non-essential components or features may also be included.

As used herein, in certain embodiments, the term "comprising" may be modified to refer to "consisting essentially of" or "consisting of".

With respect to amino acid sequences in the present disclosure, even if the present disclosure describes a polypeptide or a protein "comprising" an amino acid sequence represented by a specific SEQ ID NO, a polypeptide or a protein "consisting of" an amino acid sequence represented by a specific SEQ ID NO, or a polypeptide or a protein "having" an amino acid sequence represented by a specific SEQ ID NO, it is apparent that a protein having an amino acid sequence with deletion, modification, substitution, or addition in part of the sequence may also be used in the present disclosure, as long as it has an activity identical or corresponding to that of the polypeptide or protein consisting of the amino acid sequence of the corresponding SEQ ID NO. For example, this includes cases of addition of a sequence that does not alter the function of the protein to the N-terminus and/or C-terminus of the amino acid sequence, naturally occurring mutations, silent mutations thereof, or conservative substitutions.

As used herein, the term "protein" or "polypeptide" refers to a polymer or oligomer of consecutive amino acid residues. As used herein, "polypeptide", "protein", and "peptide" may be used interchangeably with "amino acid sequence".

In some cases, an amino acid sequence exhibiting activity may be referred to as an "enzyme". In the present disclosure, unless indicated otherwise, amino acid sequences are described in an N-terminal to C-terminal direction.

As used herein, the term "recombinant" with respect to a cell, or nucleic acid, polypeptide, or vector, means that the cell, nucleic acid, polypeptide, or vector has been modified by the introduction of a heterologous nucleic acid or polypeptide, or the alteration of a native nucleic acid or polypeptide, or that the cell is derived from a cell thus modified. Therefore, for example, recombinant cells may express genes that are not found within the native (non-recombinant) form of the cells, or may express native genes that are otherwise abnormally expressed, underexpressed or not expressed at all.

As used herein, the term "isolated" refers to a substance that exists in a non-naturally occurring environment or does not exist naturally. It includes at least substantially isolating a substance (sequence, enzyme, or nucleic acid) from a substance that is naturally associated and found in nature, for example, at least one other component having a sequence, enzyme, or nucleic acid.

For example, an isolated sequence, enzyme, or nucleic acid provided herein may be provided in a form that is substantially free of one or more contaminants.

Examples of isolated substances include: i) any non-naturally occurring substance; ii) any substance (e.g., enzyme, variant, nucleic acid, protein, peptide, or cofactor) from which one, or more, or all naturally-occurring components associated in nature have been removed; iii) any substance that has been artificially modified from a substance found in nature; or iv) any substance that has been modified to alter the amount of that substance relative to other components associated in nature *(e.g.,* increase in copy number of a gene encoding a specific substance; modification of a promoter naturally linked to a gene encoding a specific substance into a highly active promoter, etc.), but are not limited thereto.

As used herein, the term "wild-type" refers to a naturally-occurring state without artificial modification. When the term "wild-type" is used in reference to a polypeptide, it refers to a naturally occurring polypeptide which does not have artificial mutations (substitutions, insertions, deletions, *etc.)* at one or more amino acid positions. Similarly, when the term "wild-type" is used in reference to a polynucleotide, it refers to a polynucleotide lacking artificial modifications (substitutions, insertions, deletions) of one or more nucleotides. However, polynucleotides encoding wild-type polypeptides are not limited to native polynucleotides and include sequences encoding any wild-type polypeptides.

As used herein, the term "parent sequence" or "backbone" refers to a reference sequence in which modification is introduced to create a modified polypeptide. That is, the parent sequence may be a starting sequence into which modifications such as substitutions, additions, and/or deletions are to be introduced. The parent sequence may be naturally-occurring or wild-type, or a variant in which one or more substitutions, insertions or deletions have occurred in the natural or wild type, or may be an artificially synthesized sequence. When the parent sequence is an amino acid sequence exhibiting activity, i.e., an amino acid sequence of an enzyme, it may be referred to as a parent enzyme.

As used herein, the term "reference sequence" refers to a sequence used to determine the position of amino acids within any amino acid sequence. By aligning any amino acid sequence with a reference sequence, the position of an amino acid within the sequence that corresponds to a specific position in the reference sequence can be determined.

As used herein, in reference to amino acid or nucleic acid sequences, the term "fragment" refers to a portion of a parent sequence. For example, it may be a polypeptide in the form in which one or more amino acids are removed from the C- or N-terminus of the parent sequence.

As used herein, the "fragment" of an enzyme may refer to a "functional fragment". The term "functional fragment" may also be referred to as an active fragment, and refers to a polypeptide that is a portion of a parent enzyme and has enzymatic activity of the parent enzyme. For example, the functional fragment of an enzyme may comprise a catalytic site of the enzyme.

A fragment of the enzyme may comprise a portion of the full length of the parent enzyme. For example, it may comprise amino acids of at least about 20%, 30%, 40%, 50%, 60%, 70%, 80%, 90%, 95%, 98%, 99% or more, and less than 100% of the full length of the parent enzyme, but is not limited thereto.

In the present disclosure, "modifying" means changing or altering. It may be altering from a naturally occurring state. In one example, an enzyme may be altered in a manner that causes it to differ from a parent sequence or a reference sequence.

In the present disclosure, a modified enzyme may be an enzyme that does not exist in nature by itself, i.e., a non-naturally occurring enzyme.

As used herein, the term "modified" refers, for example, to something that has been altered from its naturally occurring state. The modified enzyme of the present disclosure includes non-naturally occurring enzymes or naturally occurring variants. For example, the modified enzyme of the present disclosure is a modified enzyme that has not been found in nature. For example, the modified enzyme of the present disclosure may be an enzyme that does not occur spontaneously, but is not limited thereto.

As used herein, the term "modification", when used with respect to an amino acid/nucleic acid sequence, may include: substitution of an amino acid/nucleic acid residue of a parent sequence for a different amino acid/nucleic acid residue at one or more positions in the amino acid sequence; deletion of an amino acid/nucleic acid residue (or a series of amino acid/nucleic acid residues) of a parent sequence at one or more positions; insertion of an amino acid/nucleic acid residue (or a series of amino acid/nucleic acid residues) of a parent sequence at one or more positions; truncation of the amino acid sequence at the N-terminus and/or C-terminus or of the nucleic acid sequence at the 5' and/or 3' end; and any combination thereof.

As used herein, the "variant" or "modified polypeptide" of an enzyme refers to a protein having one or more amino acids different from a parent enzyme by conservative substitution and/or modification. The "variant" or "modified polypeptide" may be used interchangeably. The variant or modified polypeptide may be non-naturally occurring, but is not limited thereto.

The variant differs from the sequence of a parent enzyme by one or more modifications, for example, amino acid substitutions, deletions, and/or insertions.

Such variants may generally be identified by modifying one or more amino acids from a parent enzyme and evaluating the properties of the modified protein. That is, the ability of a variant may be enhanced, unchanged, or reduced relative to a parent enzyme.

Additionally, certain variants may include a modified polypeptide in which one or more regions, such as an N-terminal leader sequence or transmembrane domain, have been removed.

Other variants may include a variant in which a portion has been removed from the N- and/or C-terminus of the mature protein.

The term "variant" or "modified polypeptide" may be used interchangeably with terms including modification, modified protein, mutant, mutein, divergent, and variant, and is not limited as long as the terms are used to indicate mutation.

The variant may also include deletion or addition of amino acids that have minimal influence on the properties and secondary structure of a polypeptide. For example, a polypeptide may be conjugated with a signal (or leader) sequence at the N-terminus of a protein involved in the translocation of proteins co-translationally or post-translationally. Further, the polypeptide may also be conjugated with another sequence or linker to allow for identification, purification, or synthesis of the polypeptide.

As used herein, the term "conservative substitution" refers to the substitution of an amino acid with another amino acid having similar structural and/or chemical properties. Such an amino acid substitution may generally occur based on similarity in polarity, charge, solubility, hydrophobicity, hydrophilicity, and/or amphipathic nature of the residues.

Throughout the entire specification of the present disclosure, the conventional one-letter and three-letter codes for naturally occurring amino acids are used. Additionally, the amino acids mentioned herein are abbreviated according to the IUPAC-IUB nomenclature as follows:

| | |
|---|---|
| Alanine Ala, A | Arginine Arg, R |
| Asparagine Asn, N | Aspartic acid Asp, D |
| Cysteine Cys, C | Glutamic acid Glu, E |
| Glutamine Gln, Q | Glycine Gly, G |
| Histidine His, H | Isoleucine Ile, I |
| Leucine Leu, L | Lysine Lys, K |
| Methionine Met, M | Phenylalanine Phe, F |
| Proline Pro, P | Serine Ser, S |
| Threonine Thr, T | Tryptophan Trp, W |
| Tyrosine Tyr, Y | Valine Val, V |

Meanwhile, any amino acid may be described as Xaa or X.

In addition to naturally occurring amino acids, commonly accepted three-letter codes may also be used for other amino acids such as 2-aminoisobutyric acid (Aib), N-methylglycine (Sar), and α-methyl-glutamic acid.

Amino acids can generally be classified based on similarity in polarity, charge, solubility, hydrophobicity, hydrophilicity, and/or amphipathic nature of the residues. Accordingly, amino acid substitution may generally occur based on similarity in polarity, charge, solubility, hydrophobicity, hydrophilicity, and/or amphipathic nature of the residues.

For example, among the amino acids having an electrically charged side chain (electrically charged amino acids), positively charged (basic) amino acids include arginine, lysine, and histidine, and negatively charged (acidic) amino acids include glutamic acid and aspartic acid. Further, among the amino acids having an uncharged side chain (uncharged amino acids), nonpolar amino acids include glycine, alanine, valine, leucine, isoleucine, methionine, phenylalanine, tryptophan, and proline, and polar or hydrophilic amino acids include serine, threonine, cysteine, tyrosine, asparagine, and glutamine. Among the nonpolar amino acids, aromatic amino acids include phenylalanine, tryptophan, and tyrosine.

As used herein, the term "gene" means a polynucleotide that encodes a polypeptide and a polynucleotide comprising the upstream and downstream regions of the coding region. In some embodiments, a gene may have a sequence (intron) inserted between each coding region (exon).

As used herein, the term "homology" or "identity" refers to the degree of relevance between two given amino acid sequences or nucleotide sequences, and may be expressed as a percentage. The terms "homology" and "identity" may often be used interchangeably.

The sequence homology or identity of conserved polynucleotides or polypeptides may be determined by a standard alignment algorithm, and default gap penalties established by the program used may be applied. Substantially, homologous or identical sequences may generally hybridize under moderately or highly stringent conditions to the full-length of the sequence or at least about 50%, 60%, 70%, 80%, or 90% or more of the full-length. It is apparent that hybridization also includes hybridization with polynucleotides containing codons that reflect common codon usage or codon degeneracy in the polynucleotides.

Whether any two polynucleotide or polypeptide sequences have homology, similarity, or identity may be determined by, for example, a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (the GCG program package (Devereux, J. et al., Nucleic Acids Research 12:387 (1984)), BLASTP, BLASTN, and FASTA (Atschul, S. F. et al., J Mol. Biol. 215:403 (1990); Guide to Huge Computers, Martin J. Bishop, ed., Academic Press, San Diego, 1994, and CARILLO et al. (1988) SIAM J Applied Math 48:1073) are included). For example, homology, similarity, or identity may be determined using BLAST from the National Center for Biotechnology Information (NCBI) or ClustalW, but is not limited thereto.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. In summary, the GAP program may be defined as the value obtained by dividing the number of similarly aligned symbols (i.e., nucleotides or amino acids) by the total number of the symbols in the shorter of the two sequences. The default parameters for the GAP program may include: (1) a binary comparison matrix (containing a value of 1 for identity and 0 for non- identity) and the weighted comparison matrix of Gribskov et al. (1986) Nucl. Acids Res. 14:6745 as disclosed in Schwartz and Dayhoff, eds., Atlas of Protein Sequence and Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps.

Further, whether any two polynucleotide or polypeptide sequences have homology, similarity, or identity may be identified by comparing the sequences in a Southern hybridization experiment under stringent conditions as defined. Appropriate hybridization conditions defined are within the skill of the art, and may be determined by a method well known to those skilled in the art *(e.g.,* J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York). However, the method for identifying homology, similarity, or identity and the appropriate hybridization conditions are not limited thereto.

As used herein, the term "mature polypeptide" refers to a polypeptide in a form without a signal sequence or propeptide sequence. A mature protein/polypeptide/peptide may be a functional form of a protein/polypeptide/peptide. A mature polypeptide may be in a final form after translation or post-translational modification. Examples of post-translational modification include N- or C-terminal modifications, glycosylation, phosphorylation, removal of a leader sequence, *etc.,* but are not limited thereto.

As used herein, the term "nucleic acid construct" refers to a single- or doublestranded nucleic acid molecule that includes one or more regulatory sequences and is artificially synthesized, engineered to include a specific sequence in a manner that does not exist in nature, or isolated from nature.

As used herein, the term "expression" includes any step involved in the production of a polypeptide, for example, transcription, post-transcriptional modification, translation, post-translational modification, and secretion, but is not limited thereto.

As used herein, the term "expression vector" refers to a linear or circular nucleic acid molecule comprising a coding sequence and an operably linked regulatory sequence for expression.

As used herein, the term "operably linked" refers to a configuration in which a regulatory sequence is positioned at an appropriate position to regulate the expression of a coding sequence. Thus, the term "operably linked" includes attaching or linking a regulatory region of a functional domain having a known or desired activity such as a promoter, a stop codon, a signal sequence, or an enhancer region, with a target (gene or polypeptide) so as to regulate the expression, secretion, or function of the target in accordance with the known or desired activity.

As used herein, the term "cDNA" refers to a DNA sequence that can be produced by reverse transcription from a mature, spliced mRNA molecule obtainable from a eukaryotic or prokaryotic cell. A cDNA sequence lacks intron sequences that may be present in the corresponding genomic DNA. The initial primary RNA transcript is a precursor to mRNA that undergoes a series of steps including splicing to become mature spliced mRNA.

As used herein, the term "regulatory sequence" refers to a polynucleotide sequence necessary for the expression of a coding sequence. Each regulatory sequence may be native (derived from the same origin) or foreign (derived from a different gene) to the coding sequence. Examples of the regulatory sequence may include a leader sequence, a polyadenylation sequence, a propeptide sequence, a promoter, a signal peptide sequence, an operator sequence, a sequence encoding a ribosome-binding domain, and a sequence regulating the termination of transcription and translation. The minimum unit of the regulatory sequence may include a promoter and a sequence for terminating transcription and translation.

In order to describe the modified polypeptides or variants provided herein, the following nomenclature is used.

In the present disclosure, referring to a specific position in an amino acid sequence may include referring to an amino acid present or substituted at that position. Referring to an amino acid at a specific position can be described in various ways. For example, "position 003" can be described as "position 3", "amino acid 3", "the third amino acid". Additionally, when the amino acid at position 3 is arginine(R), for example, it may be described as "R3" or "Arg3".

Amino acid substitution can be expressed by describing the amino acid before substitution, the position, and the substituted amino acid in sequence. The amino acids may be expressed using the conventional one-letter and three-letter codes. For example, when serine at position 5 of a specific sequence is substituted with cysteine, it may be described as "S5C" or "Ser5Cys".

Any amino acid at a specific position may be referred to as "X". For example, X6 refers to any amino acid at position 6. Additionally, when the substituted amino acid is expressed as X, it means that it is substituted with an amino acid different from the amino acid before substitution. For example, "V6X" indicates that V at position 6 is substituted with any amino acid other than V.

Different alterations can be expressed by simultaneously describing several types of amino acids using the symbol "/" or ",". For example, substitution of an amino acid (F) at position 20 with S or C can be described interchangeably as F20S/C or F20S,C. In another example, F/S20C means that the amino acid F or S at position 20 before substitution is substituted with C.

Multiple mutations can be described using "+". For example, descriptions such as "R3C+T36C" mean that arginine, an amino acid at position 3, is substituted with cysteine and threonine, an amino acid at position 8, is substituted with cysteine.

As used herein, the term "corresponding to" refers to an amino acid residue at the position recited in a protein or peptide, or an amino acid residue that is similar, identical, or homologous to the residue recited in a protein or peptide. Identification of the amino acid at the corresponding position may be determining a specific amino acid in a sequence that references a particular sequence. As used herein, the term "corresponding region" refers to a similar or corresponding position in a related protein or a reference protein.

In the present disclosure, SEQ ID NO: 1 can be used as a reference sequence to determine the position of amino acids in any amino acid sequence.

That is, SEQ ID NO: 1 disclosed herein can be used to determine the corresponding amino acid residues in any polypeptide having DON degradation activity. Unless indicated otherwise in the present disclosure, residues of a specific amino acid sequence are numbered relative to SEQ ID NO: 1.

For example, based on the alignment of any amino acid sequence with SEQ ID NO: 1, each amino acid residue in the amino acid sequence can be numbered with reference to the position number of the amino acid residue corresponding to the amino acid residue of SEQ ID NO: 1. For example, a sequence alignment algorithm as described herein can identify the position of an amino acid or a position where modification such as substitution, insertion, or deletion occurs compared to a query sequence (also referred to as the "reference sequence").

An example of the alignment may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277), *etc.,* but the alignment is not limited thereto.

Additionally, multiple sequence alignment can be used to identify corresponding amino acid residues in otherpolypeptides having DON degradation activity. Examples of the multiple sequence alignment known in the art include programs such as MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22: 4673-4680) using their respective default parameters, but are not limited thereto.

In addition, when the relationship between enzymes diverged from a mature polypeptide of SEQ ID NO: 1 cannot be identified by traditional sequence-based comparison, other pairwise sequence comparison algorithms may be employed (Lindahl and Elofsson, 2000, J. Mol. Biol. 295: 613-615). Higher sensitivity can be achieved in sequence-based searches by using search programs that utilize probabilistic representations of polypeptide families (profiles) to search databases. For example, the PSI BLAST program can generate profiles through an iterative database search process and detect remote homologs (Atschul et al., 1997, Nucleic Acids Res. 25: 3389-3402). When the family or superfamily of a polypeptide has one or more representatives in protein structure databases, significantly higher sensitivity can be achieved. Programs such as GenTHREADER (Jones, 1999, J. Mol. Biol. 287: 797-815; McGuffin and Jones, 2003, Bioinformatics 19: 874-881) utilize information from a variety of sources, such as PSI BLAST, secondary structure prediction, structural alignment profiles, and solvation potentials, as input to a neural network that predicts the structural fold for a query sequence. Similarly, the method of Gough et al., 2000, J. Mol. Biol. 313: 903-919 may be used to align a sequence of unknown structure with the superfamily models present in the SCOP database. These alignments may be sequentially used to generate homology models for the peptides, and such models can be assessed for accuracy using a variety of tools developed for that purpose.

For proteins of known structure, several tools and resources may be used for retrieving and generating structural alignments. For example, the SCOP superfamilies of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (CE) (Shindyalov and Bourne, 1998, Protein Engineering 11: 739-747), and implementation of these algorithms can additionally be utilized to query structure databases with a structure of interest in order to discover possible structural homologs (Holm and Park, 2000, Bioinformatics 16: 566-567).

The foregoing methods are illustrative examples and are not limited thereto.

Hereinafter, the specific embodiments of the present disclosure will be described in more detail.

As used herein, "deoxynivalenol degradation activity" means catalyzing the degradation of deoxynivalenol (DON) through conversion into the form of iso-DON.

As used herein, deoxynivalenol (DON) has the chemical formula C₁₅H₂₀O₆ and a molecular weight of 296.3 g/mol (CAS No. 51481-10-8). It is a fungal toxin belonging to type B trichothecenes, which is an epoxy-sesquiterpenoid, and is also known as vomitoxin. It can be produced by fungi that cause plant diseases, among which species of the genus *Fusarium* are known producers. Upon ingestion, DON can cause symptoms such as immunosuppression, anemia, headache, nausea, and abdominal pain in humans, and feed refusal, vomiting, growth inhibition, and reproductive disorders in animals.

Meanwhile, the term "degradation" of DON may be used to refer to detoxification of DON, inactivation of DON, and decontaminating DON-induced contamination.

As used herein, the polypeptide having DON degradation activity or DON-degrading enzyme may be, for example, a glyoxalase I. In one example, it may be a glyoxalase SPG or a specialized glyoxalase (SPG), but is not limited thereto.

As used herein, the term "parent DON-degrading enzyme" refers to a DON-degrading enzyme that is modified to produce the variant or modified polypeptide of the present disclosure. Specifically, the parent DON-degrading enzyme, parent enzyme, or parent sequence may be a naturally occurring or wild-type polypeptide or a mature polypeptide thereof, and may include a variant or functional fragment thereof. However, it is not limited thereto as long as it is a polypeptide that has DON degradation activity and is capable of serving as a parent of a variant.

The parent DON-degrading enzyme provided herein may be the polypeptide of SEQ ID NO: 1, 3, or 22, but is not limited thereto. Further, the parent DON-degrading enzyme may be a polypeptide having about 85%, 90%, 95%, 96%, 97%, 98%, 95%, 96%, 97%, 98%, or 99% or more sequence identity with the polypeptide of SEQ ID NO: 1 as long as it exhibits DON degradation activity, and a polypeptide may be included in the scope of the parent DON-degrading enzyme without limitation if it has the same or equivalent activity as the polypeptide consisting of the amino acid sequence of SEQ ID NO: 1, 3, or 22.

The parent DON-degrading enzyme of the variant provided herein may be derived from the genus *Gossypium, Handroanthus,* or *Hibiscus.* Specifically, it may be derived from the genus *Gossypium.*

As used herein, the "modified polypeptide having DON degradation activity" may be a variant of a parent DON-degrading enzyme.

As used herein, the "variant of a parent DON-degrading enzyme" or "modified polypeptide having DON degradation activity" refers to a protein having DON degradation activity and in which one or more amino acids differ from the amino acid sequence of the parent DON-degrading enzyme.

The terms "modified polypeptide having DON degradation activity", "variant of a parent DON-degrading enzyme", and "DON-degrading enzyme variant" can be used interchangeably.

The modified polypeptide provided herein may have DON degradation activity while comprising modifications of one or more amino acids in the sequence of a parent DON-degrading enzyme. The modification may be amino acid substitution and/or disulfide bond formation.

Additionally, i) the modified polypeptide has a sequence identity of 80% or more and less than 100% with SEQ ID NO: 1; and/or ii) the modified polypeptide is a polypeptide encoded by a polynucleotide having a sequence identity of 80% or more and less than 100% with a sequence encoding a mature polypeptide of SEQ ID NO: 1; and or iii) the modified polypeptide is a polypeptide encoded by a polynucleotide that hybridizes to (a) a mature polypeptide coding sequence of SEQ ID NO: 1, (b) a cDNA thereof, or (c) the full-length complement of (a) or (b) under low stringency conditions, medium stringency conditions, medium-high stringency conditions, high stringency conditions, or very high stringency conditions; and/or iv) the modified polypeptide is a functional fragment of a polypeptide of i), ii), or iii) having DON degradation activity, and the modified polypeptide may be a modified polypeptide comprising substitution of the amino acid at a position corresponding to position 65 or 121 of the polypeptide of SEQ ID NO: 1 with another amino acid.

Specifically, the variant provided herein may have DON degradation activity and include one or more amino acid modifications in the sequence of a parent DON-degrading enzyme, thereby exhibiting one or more altered functions or properties compared to the parent DON-degrading enzyme.

In one embodiment, the variant provided herein may have DON degradation activity and include one or more amino acid modifications in the sequence of a parent DON-degrading enzyme, thereby exhibiting one or more altered functions or properties compared to the parent DON-degrading enzyme, and may have one or more conserved substitutions.

The variant provided herein is a variant of a parent DON-degrading enzyme and may be a polypeptide having DON degradation activity.

In one embodiment, the variant provided herein may comprise, in the sequence of SEQ ID NO: 1 or a sequence having a sequence identity of 80% or more and less than 100% with SEQ ID NO: 1, modification at a position corresponding to position 65 or 121 of SEQ ID NO: 1.

In one embodiment, the sequence of SEQ ID NO: 1 or a sequence having a sequence identity of 80% or more and less than 100% with SEQ ID NO: 1 provided herein may be SEQ ID NO: 3 or SEQ ID NO: 22, but is not limited thereto. As used herein, the position number corresponds to the position of the polypeptide of SEQ ID NO: 1, and the term "corresponding" is as described above.

In one embodiment, the variant provided herein may comprise substitution of an amino acid at a position corresponding to position 65 or 121 of SEQ ID NO: 1, but is not limited thereto. In one embodiment of the foregoing embodiments, in the modified polypeptide provided herein, the amino acid at a position corresponding to position 65 of SEQ ID NO: 1 may be G, A, V, L, I, M, F, W, P, S, T, C, Y, N, Q, D, E, K, R, or H. Specifically, it may be M.

In one embodiment of the foregoing embodiments, the modified polypeptide provided herein may comprise substitution of the amino acid at a position corresponding to position 65 of SEQ ID NO: 1 with a nonpolar amino acid.

Additionally, in one embodiment of the foregoing embodiments, the modified polypeptide provided herein may comprise substitution of the amino acid at a position corresponding to position 65 of SEQ ID NO: 1 with methionine (M).

In one embodiment of the foregoing embodiments, in the modified polypeptide provided herein, the amino acid at a position corresponding to position 121 of SEQ ID NO: 1 may be G, A, V, L, I, M, F, W, P, S, T, C, Y, N, Q, D, E, K, R, or H. Specifically, it may be I.

In one embodiment of the foregoing embodiments, the modified polypeptide provided herein may be one in which the amino acid at the position corresponding to position 121 of SEQ ID NO: 1 is substituted with a nonpolar amino acid.

Additionally, in one embodiment of the foregoing embodiments, the modified polypeptide provided herein may comprise substitution of the amino acid at a position corresponding to position 121 of SEQ ID NO: 1 with isoleucine (I).

Further, in one embodiment of the foregoing embodiments, the variant provided herein may comprise, in the sequence of SEQ ID NO: 1 or a sequence having sequence identity of 80% or more and less than 100% with SEQ ID NO: 1, substitution of an amino acid at a position corresponding to position 65 of SEQ ID NO: 1 or substitution of an amino acid at a position corresponding to position 121of SEQ ID NO: 1. In one embodiment, it may comprise a F65M or R121I mutation in SEQ ID NO: 1 or SEQ ID NO: 3. In another embodiment, it may comprise F64M or R120I in SEQ ID NO: 22. Additionally, the variant provided herein may comprise one or more selected from SEQ ID NO: 5, SEQ ID NO: 7, SEQ ID NO: 9, SEQ ID NO: 11, SEQ ID NO: 24, and SEQ ID NO: 26, but is not limited thereto.

In one embodiment, the modified polypeptide provided herein may have a sequence identity of about 80% or more, for example, 80% or more, 85% or more, 90% or more, 91% or more, 92% or more, 93% or more, 94% or more, 95% or more, 96% or more, 97% or more, 98% or more, or 99% or more, and less than 100% with a parent DON-degrading enzyme, a mature polypeptide thereof, or a functional fragment thereof.

In one embodiment, the modified polypeptide provided herein may have a sequence identity of about 80% or more, for example, 80% or more, 85% or more, 90% or more, 91% or more, 92% or more, 93% or more, 94% or more, 95% or more, 96% or more, 97% or more, 98% or more, or 99% or more, and less than 100% with SEQ ID NO: 1.

In one embodiment, the modified polypeptide provided herein may be a polypeptide encoded by a polynucleotide having a sequence identity of about 80% or more, for example, 80% or more, 85% or more, 90% or more, 91% or more, 92% or more, 93% or more, 94% or more, 95% or more, 96% or more, 97% or more, 98% or more, or 99% or more, and less than 100% with a sequence encoding a mature polypeptide of SEQ ID NO: 1.

In one embodiment, the modified polypeptide provided herein may be a polypeptide encoded by a polynucleotide that hybridizes to (a) a mature polypeptide coding sequence of SEQ ID NO: 1, (b) a cDNA thereof, or (c) the full-length complement of (a) or (b) under low stringency conditions, medium stringency conditions, medium-high stringency conditions, high stringency conditions, or very high stringency conditions.

In one embodiment, the modified polypeptide provided herein may have a sequence identity of about 80% or more, for example, 80% or more, 85% or more, 90% or more, 91% or more, 92% or more, 93% or more, 94% or more, 95% or more, 96% or more, 97% or more, 98% or more, or 99% or more, and less than 100% with a functional fragment of SEQ ID NO: 1.

In one embodiment, the modified polypeptide provided herein may be a polypeptide encoded by a polynucleotide having a sequence identity of about 80% or more, for example, 80% or more, 85% or more, 90% or more, 91% or more, 92% or more, 93% or more, 94% or more, 95% or more, 96% or more, 97% or more, 98% or more, or 99% or more, and less than 100% with a nucleic acid base sequence represented by SEQ ID NO: 2.

In one embodiment, the modified polypeptide provided herein may comprise one or more selected from SEQ ID NOS: 5, 7, 9, 11, 24, and 26.

In one embodiment, the modified polypeptide provided herein may have, in an amino acid sequence of any one of SEQ ID NOS: 1 and 3, substitution of one or more amino acids at positions corresponding to positions 65 and 121 with other amino acids or may have, in an amino acid sequence of SEQ ID NO: 22, substitution of one or more amino acids at positions corresponding to positions 64 and 120, and it may be a polypeptide having a sequence identity of about 80% or more, for example, 80% or more, 85% or more, 90% or more, 91% or more, 92% or more, 93% or more, 94% or more, 95% or more, 96% or more, 97% or more, 98% or more, 99% or more, 97% or more, 98% or more, or 99% or more, and less than 100% with the amino acid sequence of the above-mentioned SEQ ID NO, a mature polypeptide thereof, or a functional fragment thereof.

The modified polypeptide provided herein may exhibit alterations in one or more selectable or detectable properties or attributes of a polypeptide as compared to other DON-degrading enzymes, such as wild-type DON-degrading enzymes, parent DON-degrading enzymes, and other modified polypeptides having DON degradation activity.

The properties or attributes include oxidative stability, substrate specificity, catalytic activity, thermal stability, alkaline stability, pH activity profile, resistance to proteolytic degradation, Km, k_{cat}, k_{cat}/Km ratio, protein folding, immune response induction, ability to bind to a ligand, ability to bind to a receptor, ability to be secreted, ability to be displayed on the surface of a cell, ability to oligomerize, ability to send a signal, ability to stimulate cell proliferation, ability to inhibit cell proliferation, ability to induce apoptosis, ability to be modified by phosphorylation or glycosylation, and/or ability to treat diseases, *etc.,* but are not limited thereto.

In one embodiment, the modified polypeptide provided herein may have one or more improved properties selected from DON degradation activity, thermal tolerance, and thermal stability compared to a parent sequence.

As used herein, "enzymatic activity" or "DON degradation activity" represents at least one catalytic activity. Specifically, it may be the conversion efficiency of an enzyme mainly expressed as k_{cat}/Km, but is not limited thereto.

k_{cat} refers to the catalytic constant for the rate at which a single enzyme converts substrates into products per unit time when the enzyme is fully saturated with the substrates, and is also called the turnover number. Km refers to the substrate concentration at which the reaction rate is at half the maximum value (Vmax).

Examples of the methods of expressing enzymatic activity include specific activity (µmol of converted substrate x mg ⁻¹ x min ⁻¹) or volumetric activity (µmol of converted substrate x mL ⁻¹ x min ⁻¹). However, the definition of enzymatic activity is not limited to the description above and may be defined and evaluated based on the information known in the art.

In one embodiment, the modified polypeptide provided herein may have increased enzymatic activity by about 100%, about 110%, about 120%, about 130%, about 140%, about 150%, about 160%, about 170%, about 180%, about 190%, or about 200% or more compared to the parent enzyme.

As used herein, the term "specific activity" refers to the activity of an enzyme per unit weight of protein, and can be expressed as unit/mg. The quantification of protein can be performed using, for example, SDS-PAGE or Bradford assay.

As used herein, enzyme stability means that enzymatic activity is retained during storage or reaction time. In order to measure such changes in stability, the initial enzymatic activity can be measured and compared under defined conditions at time zero (100%) and after a certain period of time (x %), and thus, the level at which the enzymatic activity is lost or enzyme stability can be expressed.

Factors that affect enzymatic activity include, for example, pH, heat, the presence of other substances (e.g., oxidizing agents, chelating agents), *etc.*

As used herein, the term "thermal stability" refers to the ability of a protein to function in a specific temperature range. In one embodiment, the polypeptide provided herein may be active in a range of about 20°C to about 120°C, and specifically in a range of about 60°C to about 100°C. In one embodiment of the foregoing embodiments, the polypeptide having DON degradation activity provided herein may be active at 50°C to 80°C, but the activity is not limited thereto.

As used herein, the term "thermal tolerance" refers to the ability of a protein to function after exposure to a specific temperature, e.g., high heat or cryogenic temperature. For example, proteins with thermal tolerance may not function at non-optimal temperatures, but may regain their function upon returning to optimal temperature conditions.

Increased stability includes an expanded range of pH, temperature, and/or duration, *etc.,* in which a protein retains its function or maintains high enzymatic activity, as compared to other enzymes such as wild-type enzymes, parent enzymes, and/or other modified polypeptides.

In the present disclosure, an increase in stability may be evaluated based on a parent DON-degrading enzyme, for example, based on a DON-degrading enzyme with the sequence of SEQ ID NO: 1 or a sequence having a sequence identity of 80% or more and less than 100% with SEQ ID NO: 1, but is not limited thereto.

In one embodiment, the modified polypeptide may have increased thermal tolerance and/or thermal stability compared to a polypeptide consisting of the amino acid sequence of SEQ ID NO: 1.

In one embodiment of the foregoing embodiments, the modified polypeptide may comprise one or more substitutions selected from the following: But it is not limited thereto.

In one embodiment of the foregoing embodiments, the properties of the modified polypeptide having DON degradation activity provided herein may be activities or improved activities suitable for applications in various industries including feed, baking, and pulp bleaching.

Another aspect of the present disclosure provides a polynucleotide encoding the modified polypeptide having DON degradation activity of the present disclosure.

As used herein, the term "polynucleotide" refers to a DNA or RNA strand of at least a certain length and is a polymer of nucleotides, in which nucleotide monomers are connected in a long chain by covalent bonds.

The polynucleotide encoding the modified polypeptide having DON degradation activity of the present disclosure includes, without limitation, any polynucleotide as long as it encodes: the modified polypeptide, the polypeptide of SEQ ID NO: 5, SEQ ID NO: 7, SEQ ID NO: 9, SEQ ID NO: 11, SEQ ID NO: 24, or SEQ ID NO: 26, or a polypeptide having activity corresponding thereto.

The polynucleotide encoding the modified polypeptide having DON degradation activity of the present disclosure may undergo various modifications in the coding region within the scope that does not change the amino acid sequence of the polypeptide, due to codon degeneracy or in consideration of the codons preferred in an organism in which the modified polypeptide is to be expressed.

For example, the polynucleotide encoding the modified polypeptide having DON degradation activity of the present disclosure may be a polynucleotide sequence encoding the amino acid sequence of SEQ ID NO: 5, SEQ ID NO: 7, SEQ ID NO: 9, SEQ ID NO: 11, SEQ ID NO: 24, or SEQ ID NO: 26, or a polypeptide having homology or identity thereto.

In one embodiment, the polynucleotide encoding the modified polypeptide having DON degradation activity of the present disclosure may have or comprise a base sequence having at least 80%, 85%, or 90% and less than 100% homology or identity to the polynucleotide sequence of SEQ ID NO: 2.

In another embodiment, the polynucleotide encoding the modified polypeptide having DON degradation activity of the present disclosure may consist of or consist essentially of a base sequence having at least 80%, 85%, 90%, 95%, 96%, 97%, 98%, or 99% or more homology or identity to the sequence of SEQ ID NO: 6, 8, 10, 12, 25, or 27. Additionally, the polynucleotide of the present disclosure may include, without limitation, a probe that can be prepared from a known gene sequence, for example, any polynucleotide sequence which may hybridize with a sequence complementary to all or part of the polynucleotide sequence of the present disclosure under stringent conditions.

The term "stringent conditions" refers to conditions that enable specific hybridization between polynucleotides. Such conditions are disclosed in detail in the literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having high homology or identity, i.e., polynucleotides having 70% or more, 75% or more, 80% or more, 85% or more, 90% or more, 95% or more, 96% or more, 97% or more, 98% or more, or 99% or more homology or identity hybridize with each other, while polynucleotides having homology or identity lower than the above homology or identity do not hybridize with each other; or may include ordinary washing conditions of Southern hybridization, i.e., washing once, specifically twice or three times, at a salt concentration and temperature corresponding to 60°C., 1×SSC, 0.1% SDS, specifically 60°C., 0.1×SSC, 0.1% SDS, and more specifically 68°C., 0.1×SSC, 0.1% SDS.

Hybridization requires that two nucleic acids have complementary sequences, although base mismatches are permissible depending on the stringency of the hybridization. The term "complementary" is used to describe a relationship between nucleotide bases that can hybridize with each other. For example, regarding DNA, adenine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

Specifically, polynucleotides having homology or identity to the polynucleotide of the present disclosure may be detected using hybridization conditions comprising a hybridization step at a Tₘ value of 55 °C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length and degree of complementarity of the polynucleotides, and these variables are well known in the art (e.g., Sambrook *et al.,* supra).

Another aspect of the present disclosure provides a vector comprising the polynucleotide encoding the modified polypeptide having DON degradation activity of the present disclosure. The modified polypeptide and polynucleotide are as described in the aspects above.

As used herein, the term "vector" refers to a DNA construct containing the nucleotide sequence of a polynucleotide encoding the target polypeptide, operably linked to an appropriate expression control region (or expression control sequence) so as to express the target polypeptide in an appropriate host. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

For example, a polynucleotide encoding a target polypeptide within a chromosome may be replaced with a modified polynucleotide through a vector for chromosomal insertion in a cell. The insertion of the polynucleotide into the chromosome may be performed by any method known in the art, such as homologous recombination, but is not limited thereto. A selection marker for confirming the chromosomal insertion may be further included. The selection marker is used to screen cells transformed via the vector, that is, to confirm the insertion of the desired nucleic acid molecule. Markers that confer selectable phenotypes, such as drug resistance, nutrient requirements, resistance to cytotoxic agents, or expression of surface proteins, may be used. In an environment treated with a selective agent, only the cells that express the selection marker survive or exhibit a distinct phenotype, allowing for the selection of transformed cells.

The vector used in the present disclosure is not particularly limited, and any vector known in the art may be used.

Examples of commonly used vectors in prokaryotic cells, as a phage vector or cosmid vector, pWE15, M13, MBL3, MBL4, IXII, ASHII, APII, t10, t11, Charon4A, and Charon21A, *etc.* may be used, and as a plasmid vector, the pBR series, pUC series, pBluescriptll series, pGEM series, pTZ series, pCL series, and pET series, etc. may be used. Specifically, vectors including pDZ, pACYC177, pACYC184, pCL, pECCG117, pUC19, pBR322, pMW118, pCC1BAC, and pDCM2 may be used.

As an example of a vector used in eukaryotic cells, a yeast expression vector may be an integrative yeast plasmid (YIp) or an extrachromosomal plasmid vector. The extrachromosomal plasmid vector may comprise a yeast episomal plasmid (YEp), a yeast replicative plasmid (YRp), or a yeast centromere plasmid (YCp). In addition, artificial yeast chromosomes (YACs) may also be used as the vector of the present disclosure. Embodiments include pESCHIS, pESC-LEU, pESC-TRP, pESC-URA, Gateway pYES-DEST52, pAO815, pGAPZ A, pGAPZ B, pGAPZ C, pGAPα A, pGAPα B, pGAPα C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NT A, pYES2/NT B, pYES2/NT C, pYES2/CT, pYES2.1, pYES-DEST52, pTEF1/Zeo, pFLD1, PichiaPinkTM, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405, and pYJ406, but are not limited thereto.

As used herein, the term "transformation" refers to the introduction of a vector containing a polynucleotide encoding a target protein into a host cell or a microorganism to enable the expression of the protein encoded by the polynucleotide within the host cell. As long as the transformed polynucleotide can be expressed within the host cell, the transformed polynucleotide may be located within the chromosome of the host cell or exist extrachromosomally. Further, the polynucleotide comprises DNA and RNA encoding the target protein. The polynucleotide may be introduced in any form, provided that it can be introduced into and expressed within a host cell. For example, the polynucleotide may be introduced into a host cell in the form of an expression cassette, which is a gene construct comprising all elements necessary for self-expression. The expression cassette may comprise a promoter operably linked to the polynucleotide, a transcription termination signal, a ribosome binding site, and a translation termination signal. The expression cassette may be in the form of a self-replicating expression vector. Further, the polynucleotide may be introduced into a host cell as-is and operably linked to a sequence necessary for expression in the host cell, but is not limited thereto.

Additionally, the term "operably linked" used herein refers to a functional linkage between a promoter sequence for initiating and mediating the transcription of the polynucleotide encoding the target polypeptide of the present disclosure, and the polynucleotide sequence.

The method for transforming the vector of the present disclosure includes any method of introducing a nucleic acid into a cell and may be performed by selecting a suitable standard technique as known in the art depending on the host cell. For example, the transformation may be carried out via electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, *etc.,* but the method is not limited thereto.

Still another aspect of the present disclosure provides a microorganism comprising one or more of: the modified polypeptide; a polynucleotide encoding the modified polypeptide; and a vector comprising the polynucleotide.

The microorganism may comprise the modified polypeptide, the polynucleotide encoding the same, a nucleic acid construct comprising the polynucleotide and/or a vector comprising the polynucleotide. The nucleic acid construct or vector may be integrated into the chromosome or may be maintained as a self-replicating extrachromosomal vector.

The microorganism of the present disclosure may include any microorganism without limitation, as long as it can express the modified polypeptide having DON degradation activity of the present disclosure. For example, the microorganism includes any cell useful in the recombinant production of a modified polypeptide having DON degradation activity of the present disclosure, for example, a prokaryotic cell or a eukaryotic cell. In another example, examples of the microorganism include fungi and bacteria.

In one embodiment, the microorganism may be of the genus *Escherichia,* for example *Escherichia coli, Escherichia albertii, Escherichia fergusonii, Escherichia hermannii, Escherichia vulneris,* or *Escherichia blattae.* In one embodiment of the foregoing embodiments, the microorganism may be *Escherichia coli,* but is not limited thereto.

Still another aspect of the present disclosure provides a composition for degrading DON, comprising one or more of: the modified polypeptide having DON degradation activity of the present disclosure; and a microorganism comprising one or more of the modified polypeptide, a polynucleotide encoding the modified polypeptide, and a vector comprising the polynucleotide.

Still another aspect of the present disclosure provides a method for degrading DON, comprising reacting DON with one or more of: the modified polypeptide having DON degradation activity of the present disclosure; and a microorganism comprising one or more of the modified polypeptide, a polynucleotide encoding the modified polypeptide, and a vector comprising the polynucleotide.

The modified polypeptide having DON degradation activity of the present disclosure and/or the microorganism comprising one or more of the modified polypeptide, a polynucleotide encoding the modified polypeptide, and a vector comprising the polynucleotide may be used to degrade substances containing DON.

In one embodiment, the modified polypeptide of the present disclosure may be used for detoxification of DON, detoxification of toxicity of DON, conversion of DON to iso-DON, and/or degradation induction through conversion of iso-DON.

Additionally, in another embodiment of the present disclosure, the polypeptide of the present disclosure may be used in the detoxification, decontamination, or degradation of toxicity (e.g. degradation) of food contaminated with DON.

In one embodiment, the composition for degrading DON provided herein may further comprise a naturally occurring substance or a non-naturally occurring substance, in addition to the modified polypeptide of the present disclosure.

In one embodiment, the composition for degrading DON provided herein may further comprise any component suitable for applications in various industries including feed, baking, biomass saccharification, and pulp bleaching.

Examples of substances that may be added include stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, *etc.,* but are not limited thereto.

Still another aspect of the present disclosure provides a feed additive composition comprising one or more of: the modified polypeptide having DON degradation activity of the present disclosure; and a microorganism comprising one or more of the modified polypeptide, a polynucleotide encoding the modified polypeptide, and a vector comprising the polynucleotide.

Still another aspect of the present disclosure provides a method for preparing a feed product, comprising mixing a feed ingredient with a feed additive composition comprising one or more of: the modified polypeptide having DON degradation activity of the present disclosure; and a microorganism comprising one or more of the modified polypeptide, a polynucleotide encoding the modified polypeptide, and a vector comprising the polynucleotide.

The microorganism comprising the modified polypeptide, a polynucleotide encoding the modified polypeptide, and/or a vector comprising the polynucleotide may be used for any one of the following applications:
a) additives in animal feed ingredients; and/or
b) animal feed supplements; and/or
c) degradation of grain-based materials *(e.g.,* whole grains or portions thereof).

The feed product of the present disclosure may refer to any natural or artificial diet, meal, or an ingredient of the meal that is intended for or suitable for consumption, ingestion, and digestion by animals, and can be prepared in various forms known in the art.

In one embodiment, the feed product may be a grain-based material (whole or partial grains or malt grains, e.g., wheat, barley, corn, oats, rye, rice, sorghum, *etc.).*

Still another aspect of the present disclosure provides a method for preparing a modified polypeptide having DON degradation activity, comprising culturing a microorganism comprising one or more of: the modified polypeptide having DON degradation activity of the present disclosure, a polynucleotide encoding the modified polypeptide, and a vector comprising the polynucleotide.

The culturing may include culturing the microorganism in a culture medium.

As used herein, the term "culturing" means that the host cell is grown under appropriately controlled environmental conditions. The culturing process of the present disclosure may be performed in a suitable medium known in the art under suitable culturing conditions known in the art. Such a culturing process may be readily adjusted and used by those skilled in the art according to the selected strain. Specifically, the culturing may be batch culturing, continuous culturing, or fed-batch culturing, but is not limited thereto.

As used herein, the term "medium" refers to a mixed substance containing nutrients required to culture the host cell as a main component, and the medium supplies nutrients, growth factors, *etc.,* including water, which are indispensable for survival and development. Specifically, any medium and culture conditions may be used for culturing the host cell without particular limitation, as long as the medium is used for the common culture of host cells. The host cell may be cultured in a common medium containing suitable carbon sources, nitrogen sources, phosphorus sources, inorganic compounds, amino acids, and/or vitamins, *etc.,* while controlling the temperature, pH, *etc.* under aerobic conditions.

In the present disclosure, the carbon sources include carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (i.e., molasses converted into reducing sugars) may be used, and other various carbon sources in appropriate amounts may be used without limitation. These carbon sources may be used alone or in combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of two or more, but are not limited thereto.

As the phosphorus sources, monobasic phosphate, dibasic potassium phosphate, or the corresponding sodium-containing salts may be used. As the inorganic compounds, sodium chloride, calcium chloride, iron chloride, magnesium sulfate, iron sulfate, manganese sulfate, calcium carbonate, *etc.* may be used. In addition, amino acids, vitamins, and/or suitable precursors, *etc.* may be included. These components or precursors may be added to the medium in a batchwise or continuous manner. However, the medium is not limited thereto.

During the culturing of the host cell, compounds such as ammonium hydroxide, potassium hydroxide, ammonia, phosphoric acid, and sulfuric acid may be added to the medium in an appropriate manner to adjust the pH of the medium. During the culturing, an antifoaming agent such as fatty acid polyglycol ester may be used to suppress foaming. To maintain an aerobic state of the medium, oxygen or oxygen-containing gas may be injected into the medium. To maintain anaerobic or microaerobic state of the medium, no gas or nitrogen, hydrogen, or carbon dioxide gas may be injected. However, the culturing conditions are not limited thereto

The temperature during the culturing of the present disclosure may be in the range from 20°C to 55°C, specifically from 25°C to 40°C, but is not limited thereto. The culturing may be carried out until a desired amount of a useful substance is obtained, and may be specifically carried out for 24 to 196 hours, but is not limited thereto.

In one embodiment, the method for preparing a modified polypeptide having DON degradation activity of the present disclosure may further comprise recovering the modified polypeptide having DON degradation activity of the present disclosure expressed during the culturing.

In another embodiment, the modified polypeptide having DON degradation activity expressed during the culturing may be recovered using a method known in the field to which the present invention pertains. For example, the modified polypeptide may be recovered from the medium through a conventional procedure including, but not limited to, collection, centrifugation, filtration, extraction, spray-drying, evaporation or precipitation.

The recovery method may be collecting the polypeptide using suitable methods known in the art depending on the culturing method of the microorganism of the present disclosure, such as batch, continuous, or fed-batch culturing methods. For example, methods such as centrifugation, filtration, treatment with a protein crystallizing precipitant (salting-out method), extraction, ultrasonic disruption, ultrafiltration, dialysis, various types of chromatography such as molecular sieve chromatography (gel filtration), adsorption chromatography, ion exchange chromatography, affinity chromatography, *etc.,* HPLC and a combination thereof may be used, and the modified polypeptide of the present disclosure can be recovered from the medium or the host cell using suitable methods known in the art.

In another embodiment, the modified polypeptide expressed by the host cell in the culturing step may not be recovered. In the foregoing embodiment, the host cell expressing the modified polypeptide may itself be used as a source of the modified polypeptide.

Still another aspect of the present disclosure provides a use of one or more of: the modified polypeptide of the present disclosure; a microorganism comprising one or more of the modified polypeptide, a polynucleotide encoding the modified polypeptide, and a vector comprising the polynucleotide, for degrading DON.

The modified polypeptide, polynucleotide, vector, microorganism, and DON degradation are as described above.

### [Mode for Carrying Out the Invention]

Hereinafter, the present disclosure will be described in more detail by way of Examples and Experimental Examples. However, these Examples and Experimental Examples are given for illustrative purposes only, and the scope of the present disclosure is not intended to be limited by these Examples and Experimental Examples.

### Example 1: Preparation of Modified Polypeptide (SPG Mutant) Having DON Degradation Activity

### Example 1-1. Construction of GbSPG-7P Mutants

A polynucleotide (SEQ ID NO: 2) encoding a SPG mutant (hereinafter referred to as GbSPG-7P; SEQ ID NO: 1) derived from *Gossypium barbadense* was cloned into a pET vector (Novagen) and used as a template.

Residues were selected to increase the activity of GbSPG-7P and primers were designed to produce two types of mutants, as shown in Table 1 (Table 1). Mutation positions based on the amino acid sequence of SEQ ID NO: 1, amino acids before and after mutation, and primer sequences are disclosed in Table 1 below in the listed order.

**[Table 1]**

| Mutant Name | Mutation | | SEQ ID NO | Primer Sequence (5'→3') |
|---|---|---|---|---|
| 7PM1 | F65M | Forward | 13 | ACGTTCCCGAGCTGAAAATGACACT TTATTTTATGGGGTACGAG |
| | | Reverse | 14 | CATTTTCAGCTCGGGAACGTCGAC CCGCTTAAGCAGACTCATG |
| 7PM2 | R121I | Forward | 15 | ATTGGATTCGGTCACATAGGGATCA CCGTGGATGATGTCAATAAG |
| | | Reverse | 16 | CCCTATGTGACCGAATCCAATTGGT TCACTATTTCCCGTGTG |

Specifically, GbSPG-7P mutants (7PM1, 7PM2) were constructed by PCR using a template (SEQ ID NO: 2), primers (SEQ ID NOS: 13 and 14 or SEQ ID NOS: 15 and 16 in Table 1) and a PCR premix (iNtRON, cat. no. 25185). PCR was carried out using an Eppendorf Mastercycler Nexus GX2, and the reaction conditions were as follows: initial denaturation at 94°C for 2 minutes;
denaturation at 94°C for 20 seconds,
annealing at 50°C for 10 seconds, and
extension at 72°C for 8 minutes (15 cycles of denaturation to extension); and
final extension at 72°C for 5 minutes.

The generated truncated mutants were ligated using a QuickChange Site-Directed Mutagenesis kit (Agilent, cat. #200518) and transformed into E. *coli* DH5α strain to confirm sequence mutations through sequencing.

### Example 1-2. Construction of GhSPG-216 Mutant Expression Vectors

Primers were designed to introduce, into corresponding positions of GhSPG-216 (SEQ ID NO: 3), a multiple mutant of SPG derived from *Gossypium harknessii* with improved thermal stability, the same mutations as those introduced into GbSPG-7P in Example 1-1, and two mutants (216M1, 216M2) were produced as shown in Table 2 below. The mutation position based on the amino acid sequence of SEQ ID NO: 3, the amino acids before and after mutation, and the primer sequences for each mutant are as disclosed in Table 2.

**[Table 2]**

| Mutant | Mutation | | SEQ ID NO | Primer Sequence (5'→3') |
|---|---|---|---|---|
| 216M1 | F65M | Forward | 17 | CCCGGATATGAAGATGACGTTATATTTTATGG GATATGAAG |
| | | Reverse | 18 | ACGTCATCTTCATATCCGGGACGTCAATTCTT TTCAAAAG |
| 216M2 | R1211 | Forward | 19 | ATCGGTTTCGGGCACATAGGCATCACGGTCG ATGACACTTAC |
| | | Reverse | 20 | CCTATGTGCCCGAAACCGATTGGTTCAGAATT GCCATTATGG |

Specifically, the expression vectors for GhSPG-216 mutants (216M1, 216M2) were constructed by PCR using a template (SEQ ID NO: 4), primers (SEQ ID NOS: 17 and 18 or SEQ ID NOS: 19 and 20 in Table 2) and a PCR premix (iNtRON, cat. no. 25185). PCR was carried out using an Eppendorf Mastercycler Nexus GX2, and the reaction conditions were as follows: initial denaturation at 94°C for 2 minutes;
denaturation at 94°C for 20 seconds,
annealing at 50°C for 10 seconds, and
extension at 72°C for 8 minutes (15 cycles of denaturation to extension); and
final extension at 72°C for 5 minutes.

The generated truncated mutants were ligated using a QuickChange Site-Directed Mutagenesis kit (Agilent, cat. #200518), and then transformed into E. *coli* DH5α strain to confirm sequence mutations through sequencing.

### Example 1-3. Construction of PgSPG-213 Expression Vector

A polynucleotide (SEQ ID NO: 23) encoding a mutant (hereinafter referred to as PgSPG-213; SEQ ID NO: 22) in which 18 mutations were introduced into SPG (hereinafter referred to as PgSPG-9; SEQ ID NO: 21) derived from *Punica granatum* was synthesized by Cosmo Genetech, cloned into pET vectors (Novagen), and used to construct expression vectors.

### Example 1-4. Construction of PgSPG-213 Mutant Expression Vectors

Primers were designed to introduce the same mutations as those introduced into GbSPG-7P in Example 1-1 into corresponding positions of GhSPG-213, and two mutants (213M1, 213M2) were produced, as shown in Table 3 below. The mutation position based on the amino acid sequence of SEQ ID NO: 22, the amino acids before and after mutation, and the primer sequences for each mutant are as disclosed in Table 3.

**[Table 3]**

| Mutant | Mutation | | SEQ ID NO | Primer Sequence (5'-3') |
|---|---|---|---|---|
| 213M1 | F64M | Forward | 28 | ACTTTCCAGAAATGAAGatgAGTCTTTATTTTATGG GTTATGAAGATAC |
| | | Reverse | 29 | catCTTCATTTCTGGAAAGTCCAGTCTTTTCAACAG GCTCATTC |
| 213M2 | R120 I | Forward | 30 | ACGGAAATAGCGAACCAAttGGCTTTGGCCACATA GGAATCACG |
| | | Reverse | 31 | aaTTGGTTCGCTATTTCCGTTGTGATATCCTTTGAA CTCCGGGTCGC |

Specifically, the expression vectors for the PgSPG-213 mutant (213M1, 213M2) were constructed by PCR using a template (SEQ ID NO: 23), primers (SEQ ID NOS: 28 and 29 or SEQ ID NOS: 30 and 31 in Table 3) and a PCR premix (iNtRON, cat. no. 25185). PCR was carried out using an Eppendorf Mastercycler Nexus GX2, and the reaction conditions were as follows: initial denaturation at 94°C for 2 minutes;
denaturation at 94°C for 20 seconds,
annealing at 50°C for 10 seconds, and
extension at 72°C for 8 minutes (15 cycles of denaturation to extension); and
final extension at 72°C for 5 minutes.

The generated truncated mutants were ligated using a QuickChange Site-Directed Mutagenesis kit (Agilent, cat. #200518), and then transformed into E. *coli* DH5α strain to confirm sequence mutations through sequencing.

### Example 1-5. Sequence Homology Among PgSPG-213, GbSPG-7P, and GhSPG-216

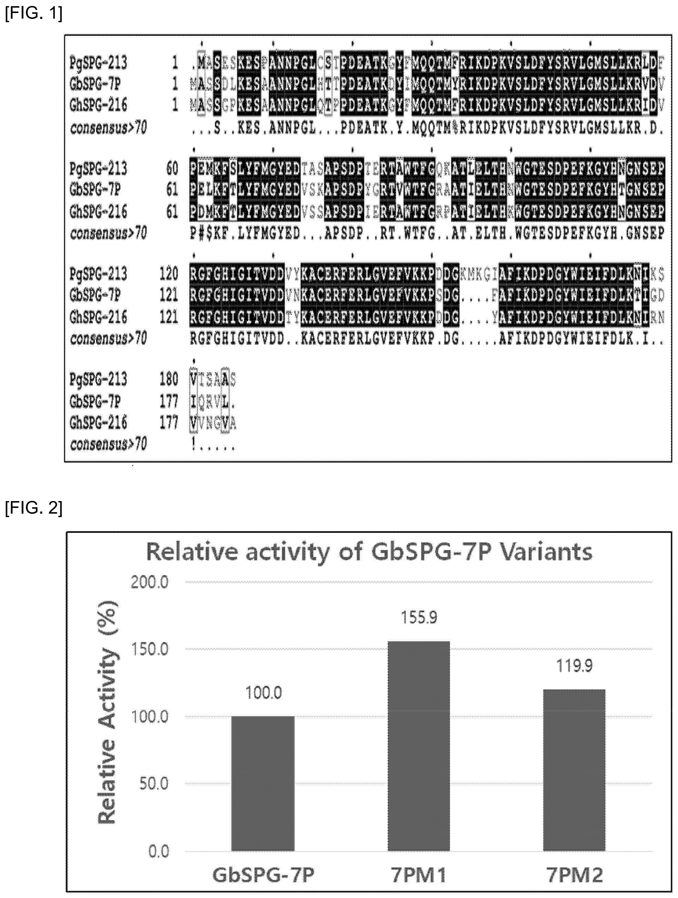

Sequence alignment (FIG. 1) among PgSPG-213, GbSPG-7P, and GhSPG-216 was generated using Clustal Omega and ESPript 3.0, and sequence homology was obtained by NCBI BLAST, which is shown in Table 4.

**[Table 4]**

| | | | |
|---|---|---|---|
| **GbSPG-7P** | 100 | | |
| **GhSPG-216** | 85.88 | 100 | |
| **PgSPG-213** | 81.01 | 83.15 | 100 |
| | **GbSPG-7P** | **GhSPG-216** | **PgSPG-213** |

As a result, it was found that homology between PgSPG-213 and GbSPG-7P was 81.01%, homology between PgSPG-213 and GhSPG-216 was 83.15%, and homology between GhSPG-216 and GbSPG-7P was 85.88%.

### Example 2: Characterization of GbSPG-7P and Mutants Thereof

### Example 2-1. Comparative Evaluation of Activity of GbSPG-7P and Mutants Thereof

Expression vectors for GbSPG-7P and mutants thereof constructed in Example 1-1 were transformed into E. *coli* BL21(DE3), which were then inoculated into sterilized LB medium (BD Difco) and pre-cultured at 37°C and 200 rpm for 16 hours. 1/100 of the culture volume was then inoculated into a flask containing sterile LB medium and cultured at 37°C and 200 rpm to an OD₆₀₀ of 0.4 to 0.5. Isopropyl β-D-1-thiogalactopyranoside (IPTG) was then added to a final concentration of 1 mM, cultured for an additional 16 hours, and the cells were recovered by centrifugation. The recovered cells were added to and resuspended in 20 mL of lysis buffer (50 mM Tris-HCl pH 8.0, 100 mM NaCl, 10 mM imidazole), and a crude enzyme solution was obtained by sonication and centrifugation. The crude enzyme solution was slowly poured onto Ni-NTA resin (Qiagen, cat. no. 30230) for adsorption, which was then washed with wash buffer (same as lysis buffer except 20 mM imidazole) and eluted with elution buffer (same as lysis buffer except 250 mM imidazole) to purify the enzyme.

Protein concentration was determined by mixing 5 µL of the diluted enzyme solution with 250 µL of Bradford solution (Quick Start^{™} Bradford 1× Dye Reagent, #5000205) and measuring the absorbance at 595 nm.

To analyze the activity of purified GhSPG-9 and GhSPG-216 mutants for DON, DON (CAS 51481-10-8) dissolved in distilled water and a purified enzyme reaction solution (25 mM Tris-HCl, 150 mM NaCl, pH 7.4) were prepared. After treatment with 500 µM of NiCl₂ as a cofactor, the reaction was carried out at 50°C for 24 hours and incubated at 100°C for 5 minutes to terminate the reaction. The amount of residual DON in the reaction mixture was measured using a High-Performance Liquid Chromatography-Ultraviolet (HPLC-UV) detector, and the ratio of DON degradation was used to measure the relative activity of the mutants. The results are as shown in Table 5 and FIG. 2.

**[Table 5]**

| **Variant** | **DON Remaining (%)** | **DON Degradation (%)** | **Relative Activity (%)** |
|---|---|---|---|
| **GbSPG-7P** | 69.14 | 30.86 | 100.00 |
| **7PM2** | 63.00 | 37.00 | 119.90 |
| **7PM1** | 51.90 | 48.10 | 155.87 |

As a result, when enzymatic activity was represented in relative ratio, it was confirmed that the activity of the two GbSPG-7P mutants (7PM1, 7PM2) was improved compared to the template GbSPG-7P by 20% to 50% or more.

### Example 2-2. Evaluation of Thermal Stability of GbSPG-7P and Mutants Thereof

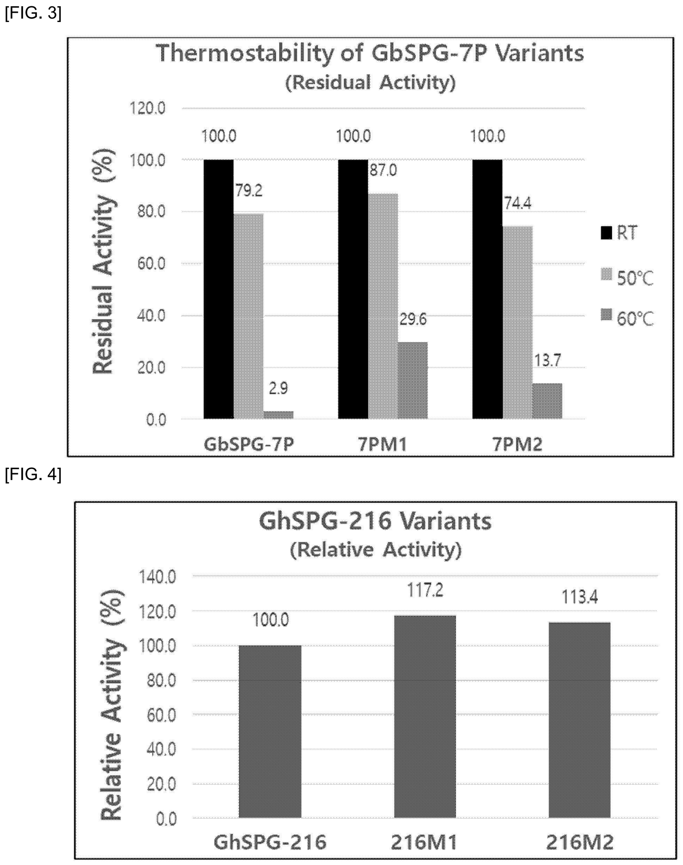

To evaluate the thermal stability of GbSPG-7P and mutants thereof obtained in Example 2-1, 50 µL of each purified enzyme was incubated at room temperature, 50°C, and 60°C for 10 minutes. Using enzyme solutions before and after heat treatment, the amount of residual DON, the degradation ratio, and the residual activity after DON degradation reaction were determined and are shown in FIG. 3. The detailed results are as shown in Table 6.

**[Table 6]**

| **Variant** | **DON Remaining (%)** | | | **DON Degradation (%)** | | | **Residual Activity (%)** | | |
|---|---|---|---|---|---|---|---|---|---|
| | **RT, 10 min.** | **50°C, 10 min.** | **60°C, 10 min.** | **RT, 10 min.** | **50°C, 10 min.** | **60°C, 10 min.** | **RT, 10 min.** | **50°C, 10 min.** | **60°C, 10 min.** |
| GbSPG-7P | 69.1 | 75.6 | 99.1 | 30.9 | 24.4 | 0.9 | 100.0 | 79.2 | 2.9 |
| GbSPG-7PM1 | 51.9 | 58.2 | 85.8 | 48.1 | 41.8 | 14.3 | 100.0 | 87.0 | 29.6 |
| GbSPG-7PM2 | 63.0 | 72.5 | 94.9 | 37.0 | 27.5 | 5.1 | 100.0 | 74.4 | 13.7 |

When residual activity was compared, it was confirmed that GbSPG-7PM1 exhibited significant increase in thermal stability compared to the template GbSPG-7P, and that GbSPG-7PM2 was at a similar level to the template at 50°C, while exhibiting an increase in residual activity of 10% or more at 60°C.

### Example 3: Characterization of GhSPG-216 and Mutants Thereof

### Example 3-1. Evaluation of Activity of GhSPG-216 and Mutants Thereof

GhSPG-216 and its two mutants (216M1, 216M2) constructed in Example 1-2 were transformed into E. *coli* BL21, which were then inoculated into sterilized LB medium (BD Difco) and pre-cultured at 37°C and 200 rpm for 16 hours. 1/100 of the culture volume was then inoculated into a flask containing sterile LB medium and cultured at 37°C and 200 rpm to an OD₆₀₀ of 0.4 to 0.5. Isopropyl β-D-1-thiogalactopyranoside (IPTG) was then added to a final concentration of 1 mM, cultured for an additional 16 hours, and the cells were recovered by centrifugation. The recovered cells were added to and resuspended in 20 mL of lysis buffer (50 mM Tris-HCl pH 8.0, 100 mM NaCl, 10 mM imidazole), and a crude enzyme solution was obtained by sonication and centrifugation. The crude enzyme solution was slowly poured onto Ni-NTA resin (Qiagen, cat. no. 30230) for adsorption, which was then washed with wash buffer (same as lysis buffer except 20 mM imidazole) and eluted with elution buffer (same as lysis buffer except 250 mM imidazole) to purify the enzyme.

Protein concentration was determined by mixing 5 µL of the diluted enzyme solution with 250 µL of Bradford solution (Quick Start^{™} Bradford 1× Dye Reagent, #5000205) and measuring the absorbance at 595 nm.

To analyze the activity of purified GhSPG-216 mutants for DON, vomitoxin (CAS 51481-10-8, deoxynivalenol) dissolved in distilled water and a purified enzyme reaction solution (25 mM Tris-HCl, 150 mM NaCl, pH 7.4) were prepared. After treatment with 500 µM of NiCl₂ as a cofactor, the reaction was carried out at 50°C for 24 hours and incubated at 100°C for 5 minutes to terminate the reaction. The amount of residual DON in the reaction mixture was measured using High-Performance Liquid Chromatography-Ultraviolet (HPLC-UV), and the ratio of DON degradation was calculated to measure relative activity. The results are as shown in Table 7 and FIG. 4.

**[Table 7]**

| **Variant** | **DON Remaining (%)** | **DON Degradation (%)** | **Relative Activity (%)** |
|---|---|---|---|
| GhSPG-216 | 49.3 | 50.7 | 100.0 |
| 216M1 | 40.5 | 59.5 | 117.2 |
| 216M2 | 42.5 | 57.6 | 113.4 |

As a result, when enzymatic activity was represented as a relative ratio, it was confirmed that the activity of the two GhSPG-216 mutants (216M1 and 216M2) was improved by 13% to 17% or more compared to the template GhSPG-216.

### Example 3-2. Evaluation of Thermal Stability of GhSPG-216 and Mutants Thereof

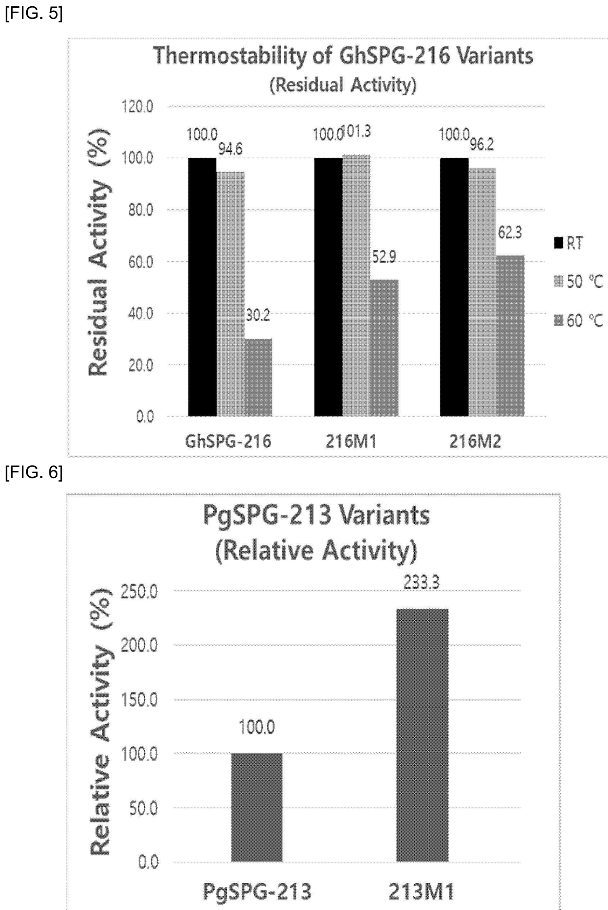

To evaluate the thermal stability of GhSPG-216 and mutants obtained in Example 3-1, 50 µL of each purified enzyme was incubated at room temperature, 50°C, and 60°C for 10 minutes. Using enzyme solutions before and after heat treatment, the amount of residual DON, the degradation ratio, and the residual activity after DON degradation reaction were determined and are shown in FIG. 5. The detailed results are as shown in Table 8.

**[Table 8]**

| **Variant** | **DON Remaining (%)** | | | **DON Degradation (%)** | | | **Residual Activity (%)** | | |
|---|---|---|---|---|---|---|---|---|---|
| | **RT, 10 min.** | **50°C, 10 min.** | **60°C, 10 min.** | **RT, 10 min.** | **50°C, 10 min.** | **60°C, 10 min.** | **RT, 10 min.** | **50°C, 10 min.** | **60°C, 10 min.** |
| GhSPG-216 | 39.94 | 43.17 | 81.89 | 60.06 | 56.83 | 18.11 | 100.0 | 94.6 | 30.2 |
| 216M1 | 42.89 | 42.13 | 69.77 | 57.11 | 57.87 | 30.23 | 100.0 | 101.3 | 52.9 |
| 216M2 | 39.52 | 41.83 | 62.30 | 60.48 | 58.17 | 37.7 | 100.0 | 96.2 | 62.3 |

When residual activity was compared, it was confirmed that both 216M1 and 216M2 were at a similar level to the template GhSPG-216 at 50°C, while the residual activity at 60°C increased by 20% to 30% or more.

### Example 4: Characterization of PgSPG-213 and Mutants Thereof

### Example 4-1. Evaluation of Activity of PgSPG-213 and Mutants Thereof

PgSPG-213 and a representative example of its mutants, 213M1, constructed in Example 1-3 were transformed into E. *coli* BL21, which were then inoculated into sterilized LB medium (BD Difco) and pre-cultured at 37°C and 200 rpm for 16 hours. 1/100 of the culture volume was then inoculated into a flask containing sterile LB medium and cultured at 37°C and 200 rpm to an OD₆₀₀ of 0.4 to 0.5. Isopropyl β-D-1-thiogalactopyranoside (IPTG) was then added to a final concentration of 1 mM, cultured for an additional 16 hours, and the cells were recovered by centrifugation. The recovered cells were added to and resuspended in 20 mL of lysis buffer (50 mM Tris-HCl pH 8.0, 100 mM NaCl, 10 mM imidazole), and a crude enzyme solution was obtained by sonication and centrifugation. The crude enzyme solution was slowly poured onto Ni-NTA resin (Qiagen, cat. no. 30230) for adsorption, which was then washed with wash buffer (same as lysis buffer except 20 mM imidazole) and eluted with elution buffer (same as lysis buffer except 250 mM imidazole) to purify the enzyme.

Protein concentration was determined by mixing 5 µL of the diluted enzyme solution with 250 µL of Bradford solution (Quick Start^{™} Bradford 1× Dye Reagent, #5000205) and measuring the absorbance at 595 nm.

To analyze the activity of purified PgSPG-213 mutant for DON, vomitoxin (CAS 51481-10-8; deoxynivalenol) dissolved in distilled water and a purified enzyme reaction solution (25 mM Tris-HCl, 150 mM NaCl, pH 7.4) were prepared. After treatment with 500 µM of NiCl₂ as a cofactor, the reaction was carried out at 50°C for 16 hours and incubated at 100°C for 5 minutes to terminate the reaction. The amount of residual DON in the reaction mixture was measured using High-Performance Liquid Chromatography-Ultraviolet (HPLC-UV), and the ratio of DON degradation was calculated to measure relative activity. The results are as shown in Table 9 and FIG. 6.

**[Table 9]**

| **Variant** | **DON Remaining (%)** | **DON Degradation (%)** | **Relative Activity (%)** |
|---|---|---|---|
| PgSPG-213 | 93.1 | 6.9 | 100.0 |
| 213M1 | 83.9 | 16.1 | 233.3 |

As a result, when enzymatic activity was represented as a relative ratio, it was confirmed that the activity of PgSPG-213 mutant (213M1) was improved compared to the template PgSPG-213 by 133% or more.

### Example 4-2. Evaluation of Thermal Stability of PgSPG-213 and Mutants Thereof

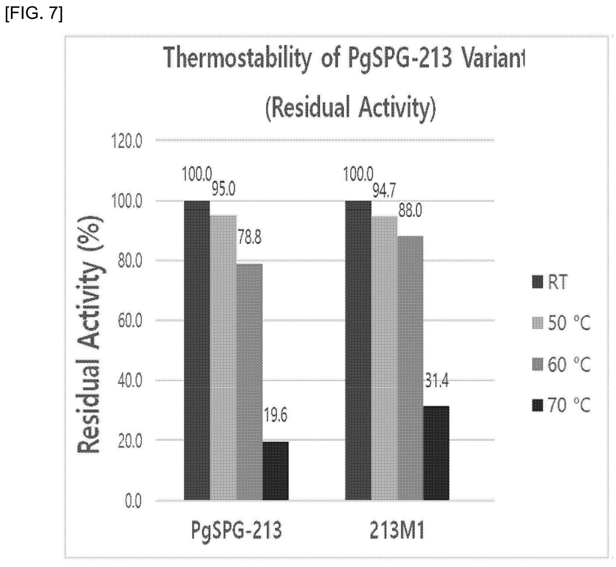

To evaluate the thermal stability of PgSPG-213 and a mutant thereof obtained in Example 4-1, 50 µL of each purified enzyme was incubated at room temperature, 50°C, and 60°C for 10 minutes. Using enzyme solutions before and after heat treatment, the amount of residual DON, the degradation ratio, and the residual activity after DON degradation reaction were determined and are shown in FIG. 7. The detailed results are as shown in Table 10.

**[Table 10]**

| **Variant** | **DON Remaining (%)** | | | | **DON Degradation (%)** | | | | **Residual Activity (%)** | | | |
|---|---|---|---|---|---|---|---|---|---|---|---|---|
| | **RT, 10 min.** | **50°C , 10 min.** | **60°C , 10 min.** | **70 ° C, 10 min.** | **RT, 10 min.** | **50°C , 10 min.** | **60°C , 10 min.** | **70 ° C, 10 min.** | **RT, 10 min.** | **50°C , 10 min.** | **60°C , 10 min.** | **70 ° C, 10 min.** |
| PgSPG-213 | 93.1 | 93.4 | 94.5 | 98.6 | 6.9 | 6.5 | 5.4 | 1.3 | 100. 0 | 95.0 | 78.8 | 19.6 |
| 213M1 | 83.9 | 84.7 | 85.7 | 94.9 | 16.1 | 15.2 | 14.2 | 5.0 | 100. 0 | 94.7 | 88.0 | 31.4 |

When residual activity was compared, it was confirmed that, at 60°C and 70°C, the mutant (213M1) exhibited an increase in residual activity of 10% or more.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. The scope of the present disclosure should be construed as including the meaning and scope of the appended claims rather than the detailed description, and all changes or variations derived from the equivalent concepts fall within the scope of the present disclosure.

## Claims

1. A modified polypeptide having deoxynivalenol (DON) degradation activity, wherein:
i) the modified polypeptide has a sequence identity of 80% or more and less than 100% with SEQ ID NO: 1; and/or
ii) the modified polypeptide is a polypeptide encoded by a polynucleotide having a sequence identity of 80% or more and less than 100% with a sequence encoding a mature polypeptide of SEQ ID NO: 1; and/or
iii) the modified polypeptide is a polypeptide encoded by a polynucleotide that hybridizes to (a) a mature polypeptide coding sequence of SEQ ID NO: 1, (b) a cDNA thereof, or (c) a full-length complement of (a) or (b), under low stringency, medium stringency, medium-high stringency, high stringency, or very high stringency conditions; and/or
iv) the modified polypeptide is a functional fragment of a polypeptide of i), ii), or iii) having DON degradation activity; and
the modified polypeptide comprises a substitution of an amino acid at position 65 or 121 with a different amino acid,
wherein the position numbers correspond to positions in the polypeptide of SEQ ID NO: 1.

2. The modified polypeptide according to claim 1, wherein the modified polypeptide comprises a substitution of the amino acid at position 121 of SEQ ID NO: 1 or SEQ ID NO: 3 with a different amino acid,
wherein the position number corresponds to a position in the polypeptide of SEQ ID NO: 1.

3. The modified polypeptide according to claim 1, wherein the modified polypeptide comprises a substitution of the amino acid at position 65 with methionine (M).

4. The modified polypeptide according to claim 1, wherein the modified polypeptide comprises a substitution of the amino acid at position 121 with isoleucine (I).

5. The modified polypeptide according to claim 1, wherein the modified polypeptide has one or more improved properties selected from DON degradation activity, thermal tolerance, and thermal stability compared to a polypeptide consisting of the amino acid sequence of SEQ ID NO: 1 or an amino acid sequence having a sequence identity of 80% or more and less than 100% with SEQ ID NO: 1.

6. The modified polypeptide according to claim 5, wherein the polypeptide consisting of an amino acid sequence having a sequence identity of 80% or more and less than 100% with SEQ ID NO: 1 is an amino acid sequence of SEQ ID NO: 3 or SEQ ID NO: 22.

7. The modified polypeptide according to claim 1, wherein the modified polypeptide comprises one or more sequences selected from SEQ ID NO: 5, SEQ ID NO: 7, SEQ ID NO: 9, SEQ ID NO: 11, SEQ ID NO: 24, and SEQ ID NO: 26.

8. A polynucleotide encoding the modified polypeptide of any one of claims 1 to 7.

9. A microorganism comprising one or more of: the modified polypeptide of claim 1; a polynucleotide encoding the modified polypeptide; and a vector comprising the polynucleotide.

10. A composition for degrading deoxynivalenol (DON), comprising one or more of: the modified polypeptide of claim 1; and a microorganism comprising one or more of the modified polypeptide, a polynucleotide encoding the modified polypeptide, and a vector comprising the polynucleotide.

11. A feed additive composition comprising one or more of: the modified polypeptide of claim 1; and a microorganism comprising one or more of the modified polypeptide, a polynucleotide encoding the modified polypeptide, and a vector comprising the polynucleotide.

12. A method for degrading deoxynivalenol (DON), comprising reacting deoxynivalenol (DON) with one or more of: the modified polypeptide of claim 1; and a microorganism comprising one or more of the modified polypeptide, a polynucleotide encoding the modified polypeptide, and a vector comprising the polynucleotide.

13. A method for preparing a feed product, comprising mixing a feed ingredient with a feed additive composition comprising one or more of: the modified polypeptide of claim 1; and a microorganism comprising one or more of the modified polypeptide, a polynucleotide encoding the modified polypeptide, and a vector comprising the polynucleotide.

14. A method for preparing a modified polypeptide having deoxynivalenol (DON) degrading activity, comprising culturing a microorganism comprising one or more of: the modified polypeptide of claim 1, a polynucleotide encoding the modified polypeptide, and a vector comprising the polynucleotide.

15. Use of one or more of: the modified polypeptide according to claim 1; a microorganism comprising one or more of the modified polypeptide, a polynucleotide encoding the modified polypeptide, and a vector comprising the polynucleotide, for degrading deoxynivalenol (DON).
